# EUROPEAN PATENT SPECIFICATION

(11) **EP 3 307 152 B1**
(45) Date of publication and mention of the grant of the patent: **31.05.2023**
(21) Application number: 16808267.5
(22) Date of filing: 09.06.2016
(51) Int. Cl.: A61N 1/04, A61N 1/39, A61N 1/36

(54) **MULTIVECTOR PATIENT CONTACT INTERFACE**
MULTIVEKTORIELLE PATIENTENKONTAKTSCHNITTSTELLE
INTERFACE MULTI-VECTORIELLE DE CONTACT AVEC LE PATIENT

(30) Priority: 10.06.2015 US 201514736180
(43) Date of publication of application: 18.04.2018
(73) Proprietor: Cardio Thrive, Inc, Concord CA 94521 (US); Mayo Foundation for Medical Education and Research, Rochester, MN 55905 (US)
(72) Inventor: SAVAGE, Walter, T., Concord, CA 94521 (US); SAVAGE, Shelley, J., Concord, CA 94521 (US); RAYMOND, Douglas, M., Livermore, CA 94550 (US); GRAY, Peter, D., Fairfield, CA 94534 (US); FRIEDMAN, Paul, A., Rochester, MN 55902 (US); ASIRVATHAM, Samuel, J., Rochester, MN 55906 (US); BRUCE, Charles, J., Rochester, MN 55902 (US); DESIMONE, Christopher, V., Rochester, MN 55901 (US)
(74) Representative: Betten & Resch
(86) International application number: PCT/US2016/036645
(87) International publication number: WO 2016/201074

(56) References cited:
- US-A1- 2001 027 270
- US-A1- 2008 200 973
- US-A1- 2014 317 914
- US-A1- 2014 371 566
- US-A1- 2014 371 567
- US-A1- 2014 371 567
- US-A1- 2014 371 806
- US-B1- 6 714 824

## Description

### Field

The disclosure relates generally to arrangements relating to medical devices. More specifically, the disclosure relates to systems used in medical device patient contact interfaces especially used in external defibrillators or wearable cardioverter defibrillators.

### Background

A primary task of the heart is to pump oxygenated, nutrient-rich blood throughout the body. Electrical impulses generated by a portion of the heart regulate the pumping cycle. When the electrical impulses follow a regular and consistent pattern, the heart functions normally and the pumping of blood is optimized. When the electrical impulses of the heart are disrupted (i.e., cardiac arrhythmia), this pattern of electrical impulses becomes chaotic or overly rapid, and a Sudden Cardiac Arrest may take place, which inhibits the circulation of blood. As a result, the brain and other critical organs are deprived of nutrients and oxygen. A person experiencing Sudden Cardiac Arrest may suddenly lose consciousness and die shortly thereafter if left untreated.

The most successful therapy for Sudden Cardiac Arrest is prompt and appropriate defibrillation. A defibrillator uses electrical shocks to restore the proper functioning of the heart. A crucial component of the success or failure of defibrillation, however, is time. Ideally, a victim should be defibrillated immediately upon suffering a Sudden Cardiac Arrest, as the victim's chances of survival dwindle rapidly for every minute without treatment.

There are a wide variety of defibrillators. For example, Implantable Cardioverter-Defibrillators (ICD) involve surgically implanting wire coils and a generator device within a person. ICDs are typically for people at high risk for a cardiac arrhythmia. When a cardiac arrhythmia is detected, a current is automatically passed through the heart of the user with little or no intervention by a third party.

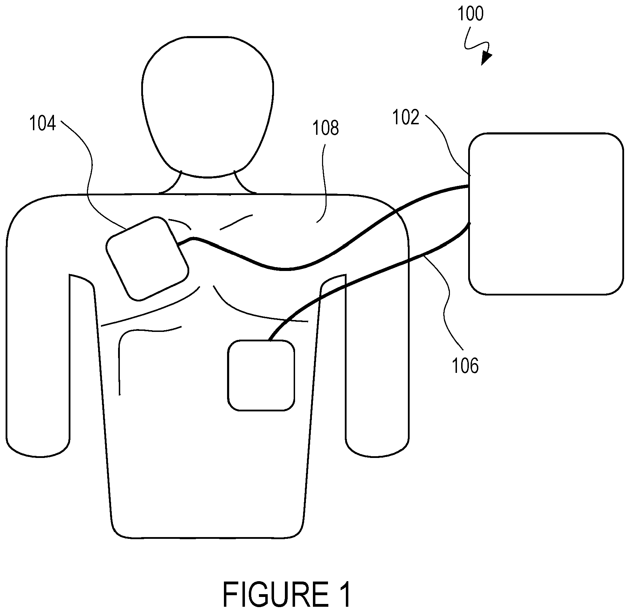

Another, more common type of defibrillator is the automated external defibrillator (AED). Rather than being implanted, the AED is an external device used by a third party to resuscitate a person who has suffered from sudden cardiac arrest. Figure 1 illustrates a conventional AED 100, which includes a base unit 102 and two pads 104. Sometimes paddles with handles are used instead of the pads 104. The pads 104 are connected to the base unit 102 using electrical cables 106.

A typical protocol for using the AED 100 is as follows. Initially, the person who has suffered from sudden cardiac arrest is placed on the floor. Clothing is removed to reveal the person's chest 108. The pads 104 are applied to appropriate locations on the chest 108, as illustrated in figure 1. The electrical system within the base unit 100 generates a high voltage between the two pads 104, which delivers an electrical shock to the person. Ideally, the shock restores a normal cardiac rhythm. In some cases, multiple shocks are required.

Although existing technologies work well, there are continuing efforts to improve the effectiveness, safety and usability of automatic external defibrillators.

Accordingly, efforts have been made to improve the availability of automated external defibrillators (AED), so that they are more likely to be in the vicinity of sudden cardiac arrest victims. Advances in medical technology have reduced the cost and size of automated external defibrillators (AED). Some modem AEDs approximate the size of a laptop computer or backpack. Even small devices may typically weigh 4-10 pounds or more. Accordingly, they are increasingly found mounted in public facilities (e.g., airports, schools, gyms, etc.) and, more rarely, residences. Unfortunately, the average success rates for cardiac resuscitation remain abysmally low (less than 8.3%).

Such solutions, while effective, are still less than ideal for most situations. Assume, for example, that a person suffers from a cardiac arrest in an airport in which multiple AEDs have been distributed. The victim's companion would nevertheless have to locate and run towards the nearest AED, pull the device off the wall, and return to the collapsed victim to render assistance. During that time, precious minutes may have passed. According to some estimates, the chance of surviving a sudden cardiac arrest is 90% if the victim is defibrillated within one minute, but declines by 10% for every minute thereafter. A defibrillator design that reduces the time to defibrillation by even two to three minutes will save more lives.

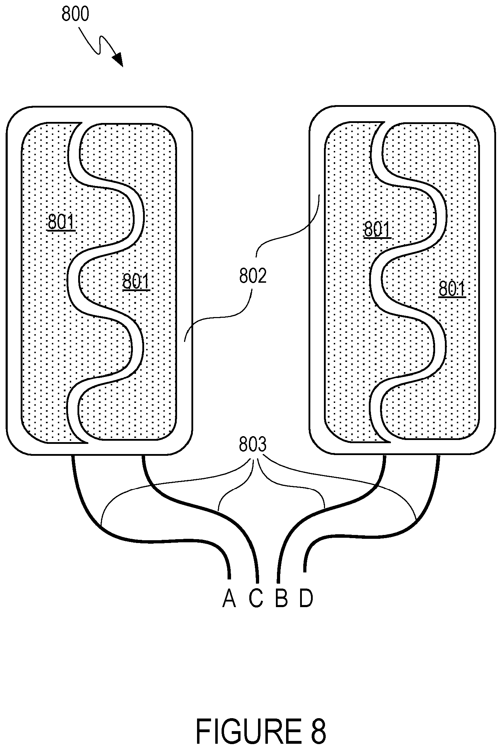

An additional challenge is that a sudden cardiac arrest may take place anywhere. People often spend time away from public facilities and their homes. For example, a sudden cardiac arrest could strike someone while biking in the hills, skiing on the mountains, strolling along the beach, or jogging on a dirt trail. Ideally, an improved AED design would be compact, light, and resistant to the elements and easily attached or detached from one's body. The typical AED design illustrated in Figure 8, which includes a sizable console or power unit whose form factor is similar to that of a laptop or backpack, seems less than ideal for the outdoors and other rigorous environments.

New and improved designs are allowing AEDs to become ultra-portable and hence to able to be easily carried by an at-risk person as they go about all of their daily activities and thus are able to be close at hand when a sudden cardiac arrest strikes outside of a hospital environment or a high traffic public area with a Public Access Defibrillator.

There are also improvements being made in the area of device usability and ease of operation for untrained bystanders. As noted above, every minute of delay or distraction can substantially decrease the victim's probability of survival. As a result, it is generally beneficial to streamline the operation of the external defibrillator so that a user of the defibrillator, who is presumably under substantial mental duress, can focus his or her attention on a few key variables.

Another type of defibrillator is the Wearable Cardioverter Defibrillator (WCD). Rather than a device being implanted into a person at-risk from Sudden Cardiac Arrest, or being used by a bystander once a person has already collapsed from experiencing a Sudden Cardiac Arrest, the WCD is an external device worn by an at-risk person which continuously monitors their heart rhythm to identify the occurrence of an arrhythmia, to then correctly identify the type of arrhythmia involved and then to automatically apply the therapeutic action required for the type of arrhythmia identified, whether this be cardioversion or defibrillation. These devices are most frequently used for patients who have been identified as potentially requiring an ICD and to effectively protect them during the two to six month medical evaluation period before a final decision is made and they are officially cleared for, or denied, an ICD.

Manual external defibrillators and WCDs are also used for external cardioversion, which is where a shaped electrical pulse is used to terminate atrial fibrillation in a patient. This also requires the use of external electrode pads.

External Defibrillators and Automated External Defibrillators on the market today make use of either rigid paddles that must be held in place on the patient's body or else flexible electrode pads (made of conductive foil and foam) which are stuck to the patient's skin. The current external defibrillators that have rigid paddle bases do not conform to the curvatures of the patient's body at the locations on the body where the paddles must be placed in order to be effective. As such the operators of these devices must apply a good amount of contact force to make physical contact across the paddle's patient contact interface and must maintain this force to maximize the surface area in contact with the patient for the sensing and reading of the heart rhythm in order that the device can detect the presence of a faulty rhythm, or arrhythmia, such as Ventricular Fibrillation or Ventricular Tachycardia so as to instruct / initiate or signal the external defibrillator to deliver the life saving therapeutic defibrillation shock pulse. The operator must also continue holding the required contact force while the device delivers the chosen therapeutic action (shock or no shock).

There are medical, practical and commercial needs to make new AEDs which are smaller, potentially even flexible, and hence much more discrete in order for patients to be able to carry the devices around with them as they go about their daily lives. This means that the life saving device is always with them for a bystander to use immediately if they drop from a Sudden Cardiac Arrest. This is far preferable to the current system of having a few AEDs mounted on the walls of a limited number of the most high traffic public locations.

Wearable Cardioverter Defibrillators on the market today are still bulky and uncomfortable for the patients to wear. They utilize a single source of energy in a box that attaches to the wearable garment (containing the sensors and the electrodes) and the energy source box normally rides on the hip. These are heavy and uncomfortable to wear and a frequent source of complaints from patients.

Current Wearable Cardioverter Defibrillators have fixed flat surface electrodes and fixed curved surface electrodes for positioning on the patient's back and abdomen. This requires that each patient has to be specially fitted for their own unit, which is time consuming for the patient. Given the limited range of device sizes available it also requires that the device be worn tightly in order to maintain a constant contact pressure with both the sensors and the electrodes, which is restrictive and can be uncomfortable for the patient. This is also the reason why the devices also employ the use of liquid conductive hydrogel, to ensure that the electrode-to-patient contact impedance is minimized. This is messy to clean up after each use when deployed by the device, and naturally this can adversely impact the patient's clothing. It also requires that the liquid reservoirs be recharged before the device can be effectively used again.

There are medical, practical and commercial needs to make new WCDs smaller and more flexible, more comfortable and more discrete for patients to wear as they go about their daily lives.

One of the major shortcomings of existing electrode pads and paddles are that they only enable a single path of the defibrillation shock, known as a shock vector, across the heart. The placement of the electrodes is known to affect the transmyocardial current. Defibrillation success depends on delivering sufficient peak transmyocardial current in order to depolarize a critical myocardial mass (thought to be in the range of 72-80% of ventricular mass). The responsiveness of individual cardiac fibers and myocytes to the electrical pulse is also thought to be linked to the physical alignment, within 3 dimensions, of the cardiac fibers and myocytes compared to the vector of the therapeutic electrical pulse.

US 2014/371567 A1 discloses that a multipart, non-uniform patient contact interface and method of use are disclosed.

US 2001/027270 A1 discloses a bio-electic interface adapter with at least two large area defibrilation electrodes and sufficient smaller precordial electrodes present therein to allow standard twelve lead ECG monitoring. The defibrillation electrodes can be affixed to the bioelectric interface via perforations, or by a functionally similar approach, to allow easy detachment in use. The bioelectric interface adaptor preferably has an undulated outer edge geometry, and provides electrodes for forming limb leads, optionally allowing use of the defibrillation electrodes as Right Arm and Left Leg electrodes. Limb lead forming electrodes are preferable affixed via perforations, or by a functionally similar approach, to allow easy detachment and deployment to conventional limb electrode locations, in use.

US 2008/200973 A1 discloses that in accordance with embodiments of the present technique an electrode pad for medical use is provides. The electrode pad comprises a support layer a plurality of electrodes mounted on the support layer and electrically insulated from one another, and a plurality of leads electrically coupled to the electrodes for selectively placing the electrodes at a desired electrical potential.

US 6 714 824 B1 discloses that electrodes are capable of delivering synchronized cardioversion and pacing energy pulses as well as defibrillation energy pulses to a patient. The electrodes are also appropriate for use with an automatic or semi-automatic external defibrillator (AED) as well as defibrillators capable of cardioversion and manual defibrillators. The electrodes have a plurality of conductors sized for delivering electrotherapy to patients of different sizes. Preferably, conductors can be used singly or in combination for therapy. The electrodes may also be separated to form electrodes of smaller dimension for application to a smaller patient.

### Summary of the invention

The invention is defined by independent claim 1. Preferred embodiments are defined by the dependent claims.

### Brief Description of the Drawings

Figure 1 diagrammatically illustrates an example of a conventional external defibrillator.
Figure 2 illustrates a standard pair of single shock vector electrode pads.
Figure 3 illustrates a pair of multi-pad multi-vector electrode pads.
Figure 4 illustrates a pair of split vertical multi-vector electrode pads.
Figure 5 illustrates a pair of multi-split vertical multi-vector electrode pads.
Figure 6 illustrates a pair of split diagonal multi-vector electrode pads.
Figure 7 illustrates a pair of multi-split diagonal multi-vector electrode pads.
Figure 8 illustrates a pair of split non-linear multi-vector electrode pads.
Figure 9 illustrates an example of a novel compact external defibrillator.
Figure 10 illustrates an electrode pad set up utilizing a one-electrode-to-many-electrode shock vector arrangement.
Figure 11 illustrates placement positions on a patient of some multi-pad multi-vector electrode pads.
Figure 12 illustrates placement positions on a patient of a pair of dual-pad multi-vector electrode pads.
Figure 13 illustrates electrodes and sensors built into a cap or skullcap device, not covered by the claims but helpful for understanding the present invention.
Figure 14 illustrates electrodes and sensors built into a girdle, harness or vest device, not covered by the claims but helpful for understanding the present invention.

### Detailed Description of One or More Embodiments

The presently claimed invention is defined in independent claim 1. Embodiments are defined in the dependent claims. The disclosure is particularly applicable to a multi-vector patient contact interface that may be used with an external defibrillator or an external cardioverter (for use in terminating Atrial Fibrillation or other non-lethal cardiac arrhythmias) and it is in this context that the disclosure will be described. It will be appreciated, however, that the multi-vector patient contact interface has greater utility since it may be used with any medical device or other system in which it is desirable to be able to deliver an electric or therapeutic pulse via multiple pathways, whether simultaneously or sequentially or with some greater or lesser degree of overlap in the timing of the pulse deliveries.

While the physical alignment, within 3 dimensions, of the cardiac fibers and myocytes of an actual patient is not knowable at the time of attempted rescue, if multiple shock vectors are used for/ within the same pulse then this will effectively increase the number of cells affected and depolarized and so increase the probability of a successful defibrillation of the patient. Thus, a system capable of delivering shocks across multiple shock vectors increases the probability of successful defibrillation by correcting for the potentially suboptimal vector used in an emergency situation. Furthermore ,the ability to deliver a wide variety of multiphasic shocks, across multiple vectors will introduce significant advantages into clinical use by combining novel form factors (small, always available, and with distributed shock vectors) and novel waveforms to solve the problem of readily available sources of successful defibrillation to treat Ventricular Fibrillation. This approach also provides significant clinical advantages in the form of increased efficaciousness over existing approaches for lower shock energies.

The system may have circuitry and the energy source/reservoir that may be re-distributed from the one large container/enclosure found in existing AEDs into two or more smaller containers. Each of these smaller containers has their own circuitry and energy source/reservoir and they are also connected to the defibrillation shock electrodes. The two or more smaller containers are then connected to each other electrically and packaged together for easy transportation. In the wearable AED system, the two or more smaller containers may be mounted on the body of the patient.

The use of multiple energy reservoirs allows for the generation and delivery of multiple pulses or for each phase of a multiphasic pulse to be separately generated and separately delivered. These multiple pulses can be delivered via one or more different shock vectors if the energy reservoirs are connected to a plurality of electrodes. This disclosure allows for the separate pulses or even the separate phases of a multiphasic pulse to be delivered via different shock vectors (including through completely different combinations of electrodes) in order to enhance the overall percentage of cardiac tissue that is successfully defibrillated or cardioverted and so more effectively terminate the lethal/non-lethal arrhythmia in the patient. This disclosure also allows for the static or dynamic configuration of one-to-many and many-to-one shock vector arrangements as an alternate or additional method of enhancing the overall percentage of cardiac tissue that is successfully defibrillated or cardioverted. This approach can also be used for delivering electrical pulses, at any level of energy, in other therapeutic and clinical areas outside of cardiac stimulation in order to cause a specific therapeutic outcome in a patient such as in the fields of neurological stimulation, gastrointestinal stimulation or the stimulation of specific internal organs or nerve systems within a patient's body.

The multi-vector patient contact interface may also include and employ a mix of sensor types, such as ECG sensors and LED optical pulse detectors, in addition to or combined with the therapeutic shock electrodes. This mix means that the external defibrillator's accuracy of the detection of shockable arrhythmias can be significantly improved. The mix of sensor types may further include sensors which can be active in nature, passive in nature, or a combination of the two types.

One embodiment of the multi-vector patient contact interface allows external defibrillators with rigid paddles to provide multiple conductive electrode surfaces in contact with the patient's body through the use of just the standard two paddle handles.

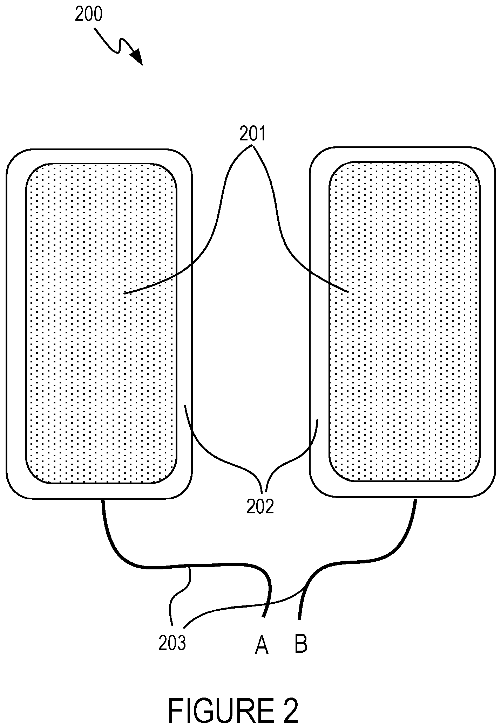

Figure 2 illustrates a pair of standard single vector electrode pads (200), used by the majority of current external defibrillators and cardioverters, exhibiting the standard adhesive and conductive electrode patient-facing surfaces, one on each of the single vector electrode pads (201) and the non-conductive adhesive mounted on the electrode pad backing material which surrounds (202) each of the adhesive and conductive electrode surfaces (201) and the electrical leads from the central defibrillator unit to each of the conductive electrode pads (203).

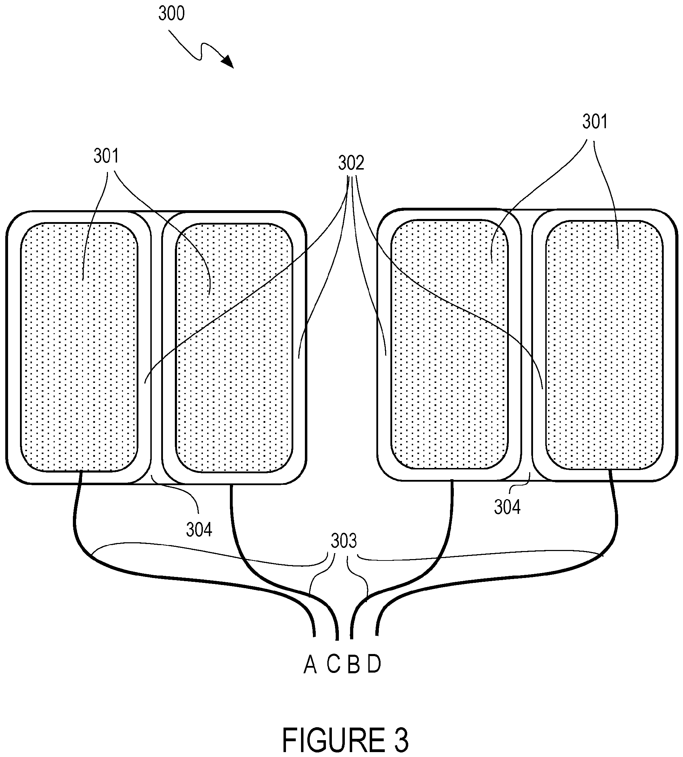

Figure 3 illustrates an embodiment with a pair of multi-pad multi-vector electrode pads (300). The multiple adhesive and conductive electrode patient-facing surfaces (301) are surrounded by areas of non-conductive adhesive (302) which are mounted on the non-conductive electrode pad backing material/substrate (304) so that each electrode patient-facing surface (301) is electrically isolated from the other electrode patient-facing surfaces. This electrode pad backing material (304) in some embodiments is able to be folded along the axis between the immediately adjacent adhesive and conductive electrode patient-facing surfaces. Each of the adhesive and conductive electrode patient-facing surfaces (301) is connected to an electrical lead (303) that connects back to the defibrillator. Each of these electrical leads is a distinctly separate electrical pathway (as shown by 303A, 303B, 303C and 303D) and they can be used separately or in tandem to deliver pulses or the separate phases of pulses in any combination of shock vectors. In some embodiments this coordination is performed by static wiring and in other embodiments it is implemented via dynamic switching within the defibrillator or within a separate module that is located between the electrode pads and the defibrillator unit. In some embodiments the electrical leads (303) are directly integrated into a housing on the non-patient facing side of the electrode pads. In some other embodiments, each of the electrode pads may have a single main adhesive and conductive electrode patient-facing surface (301) with a surrounding area of non-conductive adhesive (302) and the non-conductive electrode pad backing material (304) so that each electrode pad is separate. The number of electrode pads (whether separate or combined together as shown in Figure 3) can be any number (n) where n = 2 or larger and the number used in a given embodiment can reflect the specific purposes intended and the shock vectors desired.

In the embodiment shown in Figure 3 and Figures 4-10 described below, the electrode pad backing material/substrate may be flexible and thus capable of providing optimal contact with an uneven contact surface when the multi-vector patient contact surface is placed against the uneven contact surface. Thus, the electrode pad backing material/substrate and patient contact surface may conform to the uneven contact surface, such as a body part of a patient and the like. The electrode pad backing material/substrate may also be made of a rigid material.

In the embodiment shown in Figure 3 and Figures 4-10 described below, the conductive electrode patient-facing surface (patient contact) may be an electrode as shown in the figures. However, the conductive electrode patient-facing surface may also be a sensor that may be of a type described. In addition, in some embodiments, the patient contacts may be two or more electrodes and one or more sensors. In those embodiments, the one or more sensors may be arranged in the configuration separately from the two or more electrodes and/or the one or more sensors may be arranged in the configuration intermixed with the two or more electrodes.

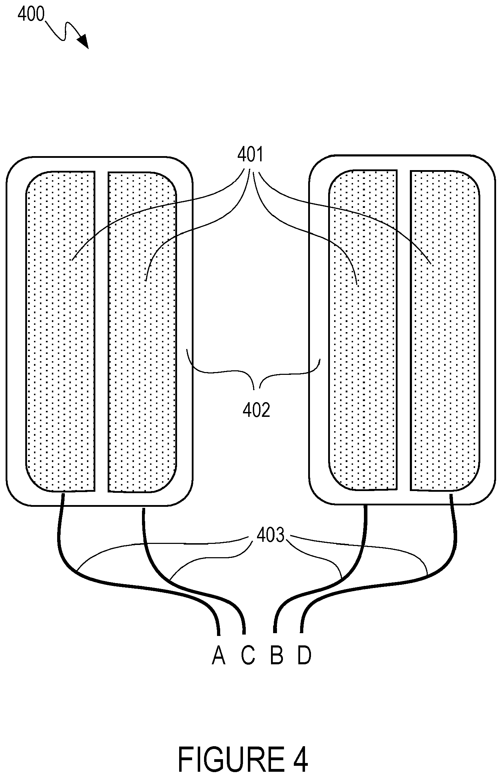

Figure 4 illustrates a pair of split vertical multi-vector electrode pads (400). The multiple adhesive and conductive electrode patient-facing surface portions (401) are surrounded by areas of non-conductive adhesive (402) which are mounted on the electrode pad backing material so that each portion 401 is electrically isolated. Each of the adhesive and conductive electrode patient-facing surface portions (401) is connected to an electrical lead (403) that connects back to the defibrillator. Each of these electrical leads is a distinctly separate electrical pathway (as shown here by 403A, 403B, 403C and 403D) and they can be used separately or coordinated in groupings to deliver pulses or the separate phases of pulses in any combination of individual or simultaneous vectors. In some embodiments this coordination is performed by static wiring and in other embodiments it is implemented via dynamic switching within the defibrillator or within a separate module that is located between the electrode pads and the defibrillator unit. In some embodiments the electrical leads (403) are directly integrated into a housing on the non-patient facing side of the electrode pads. In the example in Figure 4, four electrodes may be fit into the area of two electrode pads.

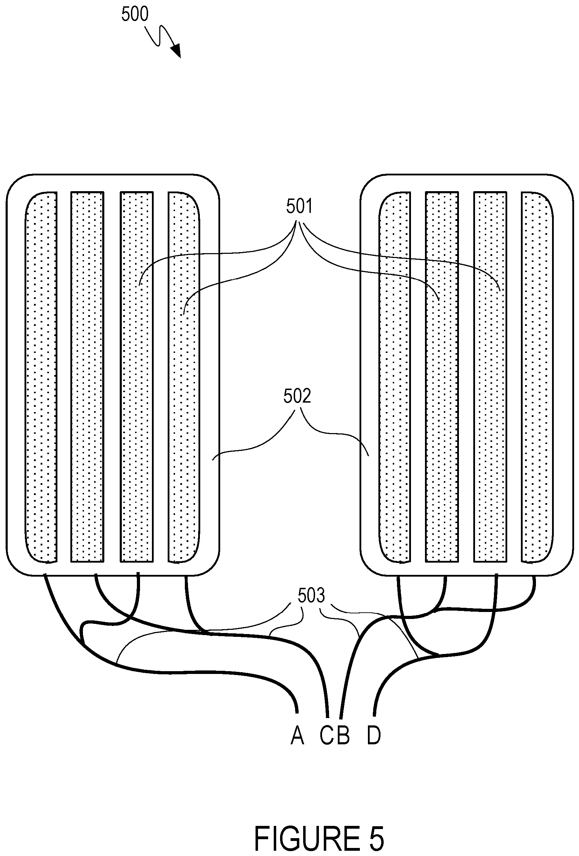

Figure 5 illustrates a pair of multi-split vertical multi-vector electrode pads (500). The multiple adhesive and conductive electrode patient-facing surface portions (501) are surrounded by areas of non-conductive adhesive (502) which are mounted on the electrode pad backing material so that each portion 501 is electrically isolated. Each of the adhesive and conductive electrode patient-facing surface portions (501) is connected to an electrical lead (503) that connects back to the defibrillator. Each of these electrical leads is a distinctly separate electrical pathway (for this embodiment they are shown grouped into pairs by 503A, 503B, 503C and 503D) and they can be used separately or in tandem to deliver pulses or the separate phases of pulses in any combination of vectors. In some embodiments this coordination/pairing of the electrical leads is performed by static wiring and in other embodiments it is implemented via dynamic switching within the defibrillator or within a separate module that is located between the electrode pads and the defibrillator unit. In some embodiments the electrical leads (503) are directly integrated into a housing on the non-patient facing side of the electrode pads.

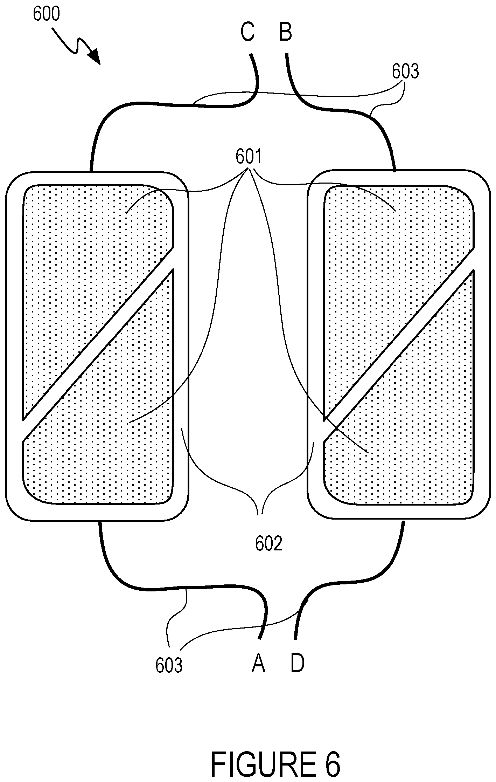

Figure 6 illustrates a pair of split diagonal multi-vector electrode pads (600). The multiple adhesive and conductive electrode patient-facing surface portions (601) are surrounded by areas of non-conductive adhesive (602) which are mounted on the electrode pad backing material so that each portion 601 is electrically isolated. Each of the adhesive and conductive electrode patient-facing surface portions (601) is connected to an electrical lead (603) that connects back to the defibrillator. Each of these electrical leads is a distinctly separate electrical pathway (as shown here by 603A, 603B, 603C and 603D) and they can be used separately or coordinated in groupings to deliver pulses or the separate phases of pulses in any combination of individual or simultaneous vectors. In some embodiments this coordination is performed by static wiring and in other embodiments it is implemented via dynamic switching within the defibrillator or within a separate module that is located between the electrode pads and the defibrillator unit. In some embodiments the electrical leads (603) are directly integrated into a housing on the non-patient facing side of the electrode pads.

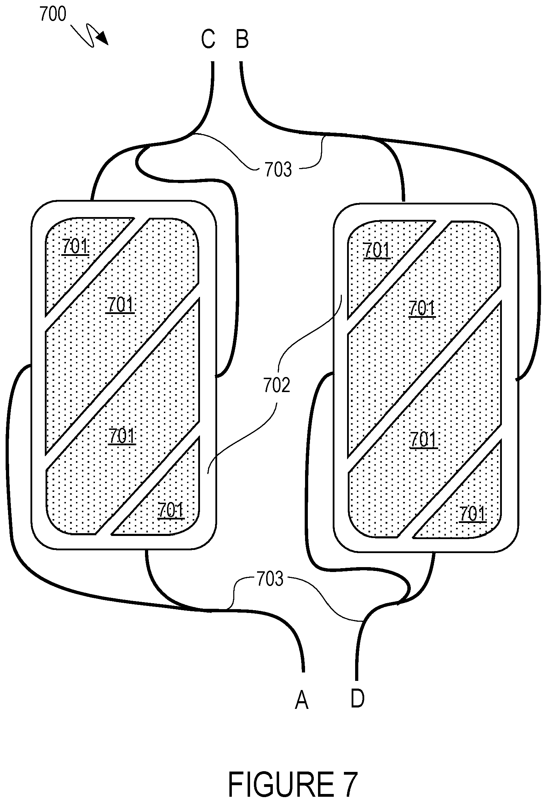

Figure 7 illustrates a pair of multi-split diagonal multi-vector electrode pads (700). The multiple adhesive and conductive electrode patient-facing surface portions (701) are surrounded by areas of non-conductive adhesive (702) which are mounted on the electrode pad backing material so that each portion 701 is electrically isolated. Each of the adhesive and conductive electrode patient-facing surface portions (701) is connected to an electrical lead (703) that connects back to the defibrillator. Each of these electrical leads is a distinctly separate electrical pathway (for this embodiment they are shown grouped into pairs by 703A, 703B, 703C and 703D) and they can be used separately or in tandem to deliver pulses or the separate phases of pulses in any combination of vectors. In some embodiments this coordination/pairing of the electrical leads is performed by static wiring and in other embodiments it is implemented via dynamic switching within the defibrillator or within a separate module that is located between the electrode pads and the defibrillator unit. In some embodiments the electrical leads (703) are directly integrated into a housing on the non-patient facing side of the electrode pads.

Although the embodiments in Figures 4-7 show conductive electrode patient-facing surface portions that are shaped and sized similarly, the device may also have conductive electrode patient-facing surface portions wherein the shape and/or size of each conductive electrode patient-facing surface portions is not uniform. Although the embodiments in Figures 4-7 disclose each conductive electrode patient-facing surface portion connected to a separate electrical lead, there may be embodiments in which two or more of the conductive electrode patient-facing surface portions may be connected to the same electrical lead, which is, however, not covered by the claims but helpful for understanding the present invention.

Figure 8 illustrates a pair of split non-linear multi-vector electrode pads (800). The multiple adhesive and conductive electrode patient-facing surface portions (801) are surrounded by areas of non-conductive adhesive (802) which are mounted on the electrode pad backing material so that each portion 801 is electrically isolated. Each of the adhesive and conductive electrode patient-facing surface portions (801) is connected to an electrical lead (803) that connects back to the defibrillator. Each of these electrical leads is a distinctly separate electrical pathway (as shown here by 803A, 803B, 803C and 803D) and they can be used separately or coordinated in groupings to deliver pulses or the separate phases of pulses in any combination of individual or simultaneous vectors. In some embodiments this coordination is performed by static wiring and in other embodiments it is implemented via dynamic switching within the defibrillator or within a separate module that is located between the electrode pads and the defibrillator unit. In some embodiments the electrical leads (803) are directly integrated into a housing on the non-patient facing side of the electrode pads.

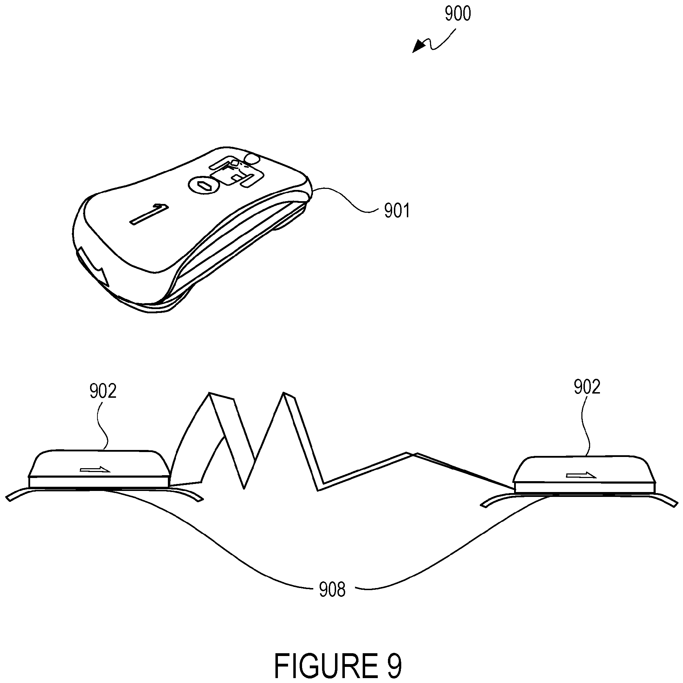

Figure 9 illustrates an example of a novel compact external defibrillator (900). The device is shown in a non deployed form (901) with the electrode pads stored internally. Upon being deployed the device assumes the form of two connected housings (902) each of which has an electrode pad on the patient-facing side (908), shown here facing down. With the device circuitry contained within the housings on the non-patient-facing sides it is plain to see that external electrical leads are not required because the electrical leads are directly integrated through the electrode pad backing material and into the housings on the non-patient facing sides of the electrode pads. In addition, as the illustration clearly shows, there is ample room for one or more of the pages/segments of the unfolding accordion (which connects the two housings/paddles together) to also contain/include additional electrode pads on the patient-facing side. Such an embodiment of the device would then allow for the delivery of shocks over multiple sequential or simultaneous shock vectors. This then allows each individual waveform phase of a multiphasic waveform pulse to be delivered over a shock vector different to the waveform phase preceding it, and hence starting from an electrode site different to that which the previous waveform phase started from. This is a very unique and important form factor enhancement, that permits small size, rapid application, and multiple vectors. Multiple additional electrodes may also be useful for enhanced sensing and rhythm detection by increasing the number of ECG sensing locations and hence the fidelity of the ECG signals received and of the ECG signal processing performed.

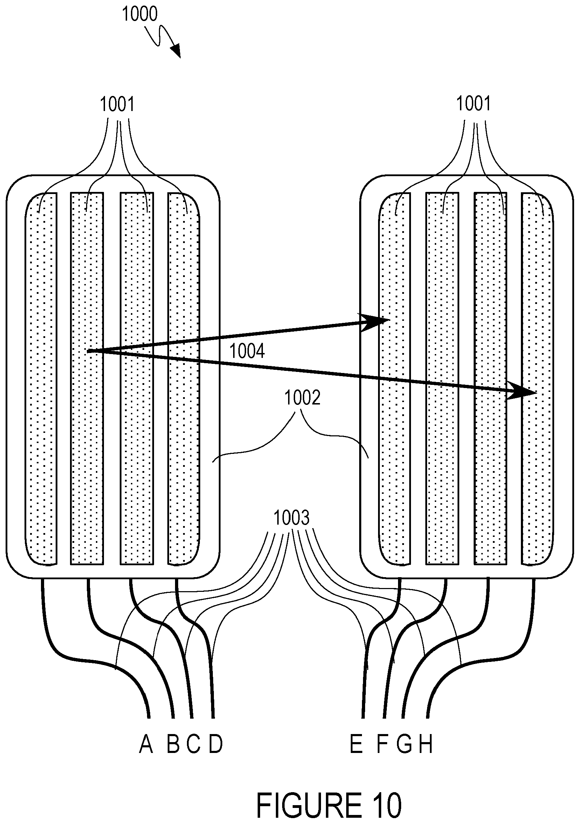

Figure 10 illustrates an example of an embodiment that may facilitate a one-to-many shock vector scenario. This illustration merely uses a single potential embodiment for the purpose of illustration, and it will be plain to those skilled in the art that there are a large range of relevant embodiments. The embodiment shown here uses a pair of multi-split vertical multi-vector electrode pads (1000) with each adhesive and conductive patient contact surface portion (1001) wired separately with an individual electrical lead back to the defibrillator (1003A, 1003B, 1003C, 1003D, 1003E, 1003F, 1003G, 1003H). These electrical leads (1003) and the adhesive and conductive patient contact surface portion (1001) can be used separately or coordinated in groupings to deliver pulses or the separate phases of pulses in any combination of individual or simultaneous vectors. In some embodiments this coordination is performed by static wiring and in other embodiments it is implemented via dynamic switching within the defibrillator or within a separate module that is located between the electrode pads and the defibrillator unit. In this example the electrical pulse or the single phase of a multiphasic pulse is delivered from a single adhesive and conductive patient contact surface portion (1001B) to the two adhesive and conductive patient contact surface portions (1001E and 1001H) via the patient upon which the electrode pads (1000) are placed. This ensures that more cells in the cardiac tissue are affected by the two simultaneous vectors of the shock (1004) than would be by using only a single vector, as two different vectors are used and the second vector will affect cardiac cells that were not oriented in the right way for the first vector to affect.

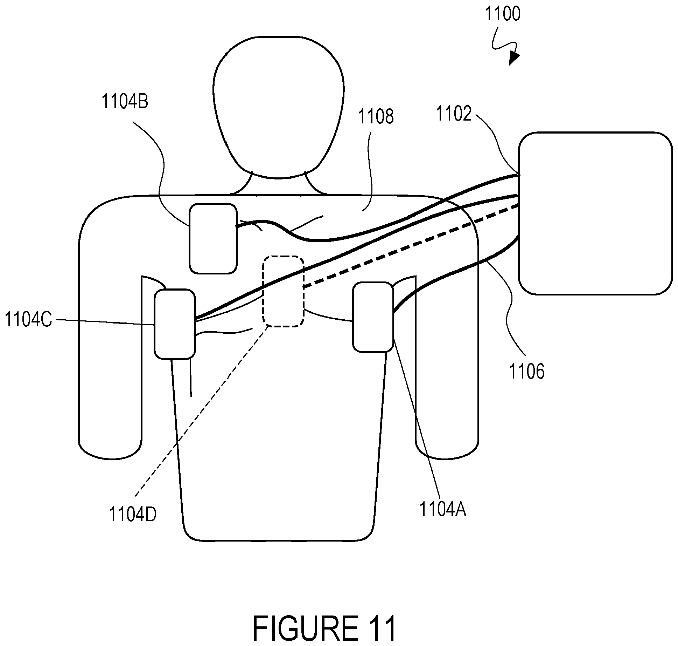

Figure 11 illustrates some of range of the potential placement positions on a patient of some multi-pad multi-vector electrode pads. The pads shown (1104A, 1104B, 1104C, 1104D) are an embodiment of the multi-pad multi-vector electrode pads shown in Figure 3, where the individual electrode pads involved are able to be separately placed and are not linked together into pairs of electrode pads via a common piece of electrode pad non-conductive backing material (304). Hence it is possible for the four pads shown in this embodiment (1104A, 1104B, 1104C, 1104D) to be each be placed in separate locations on the patient. The locations shown in Figure 11 are: on the side of the torso just under the left arm (1104A), on the front of the torso just under the right collar bone (1104B), on the side of the torso just under the right arm (1104C) and in the center of the back of the torso (1104D). Any other therapeutically efficacious location on the patient's torse, limbs or head can be used instead or in addition to the locations shown here, according to the shock vectors required. As previously stated, the shock vectors can be on a one-to-one, one-to-many, many-to-one, or many-to-many basis according to the therapeutic need or preference.

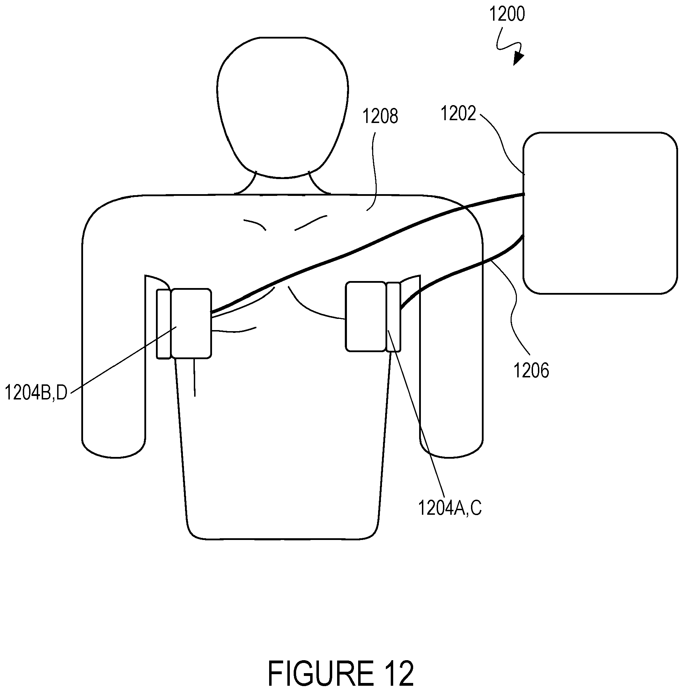

Figure 12 illustrates placement positions on a patient of a pair of dual-pad multi-vector electrode pads. The pads shown (1204 A,C and 1204 B,D) are an embodiment of the multi-pad multi-vector electrode pads shown in Figure 3, where the individual electrode pads involved are linked together in pairs and joined by a common piece of electrode pad non-conductive backing material (304). Hence it is possible for the two pairs of pads shown in this embodiment (1204 A,C and 1204 B,D) to be each placed in separate locations on the patient. The locations shown in Figure 12 are: on the side of the torso just under the left arm (1204 A,C), and on the side of the torso just under the right arm (1204 B,D). Any other therapeutically efficacious location on the patient's torse, limbs or head can be used instead or in addition to the locations shown here, according to the shock vectors required. As previously stated, the shock vectors can be on a one-to-one, one-to-many, many-to-one, or many-to-many basis according to the therapeutic need or clinical preference. In a preferred embodiment these multi-vector electrode pads are integrated into an ECG sensing garment, such as a wearable shirt. Such ECG sensing garments generally utilize capacitively coupled sensing electrodes and provide a form factor that is optimal for the inclusion of multi-vector shock electrodes, which are positioned all around the wearers torso in order to optimize the exposure of the heart to a defibrillating waveform. This approach along with small distributed electronics and power sources lends itself well to the wearable form factor.

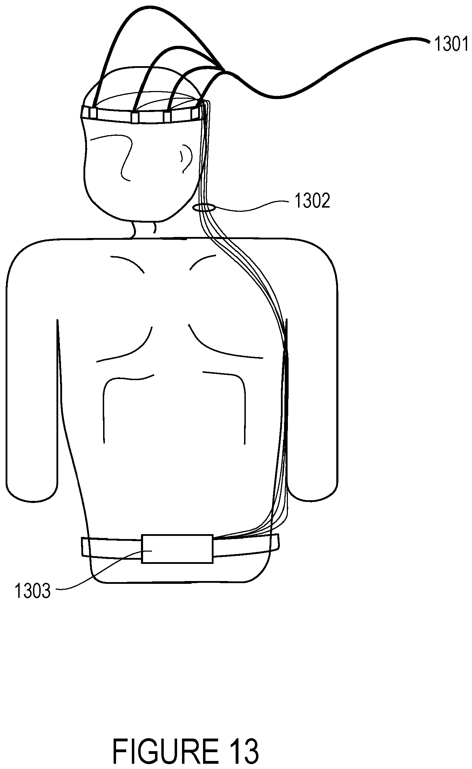

Figure 13 illustrates an embodiment of the system, which is not covered by the claims but helpful for understanding the present invention, and which is purposed to the cranial or neurological stimulation of a patient. The plurality of stimulation electrodes 1301 are shown here on a circumferential headband or strap. In other possible embodiments these stimulation electrodes 1301 can just as easily be incorporated into a cap, helmet or skull cap configuration which places the electrodes into more than just a single two dimensional plane of alignment. Such systems can be used for the control and/or termination of seizures, pain, migraine, and tremors amongst other clinical uses. The electrical leads 1302 are shown here linking the head mounted electrode system to the belt mounted pulse generaton and control electronics 1303. In other embodiments the pulse generation and control electronics 1303 are mounted on the upper arm of the patient, as well as being incorporated directly into the head mounted system. In the first of these alternate embodiments the length of the electrical leads 1302 can be significantly shortened, and in the second of these alternate embodiments the electrical leads 1302 would be internal to the head mounted system. In all possible embodiments the electrodes can be dual stimulation and sensing electrodes in addition to purely stimulation electrodes, or a mixture of the two types.

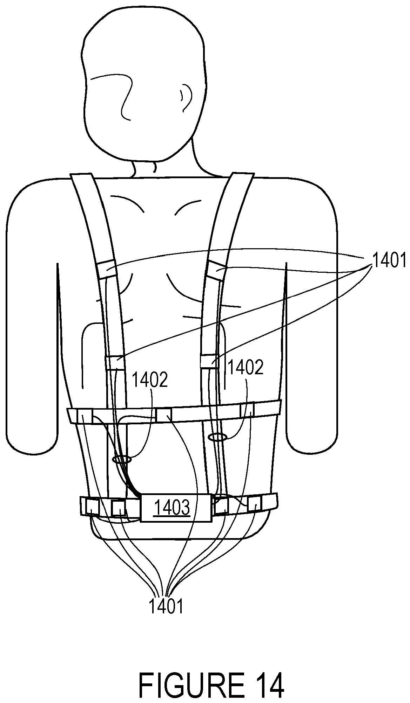

Figure 14 illustrates an embodiment of the system, which is not covered bv the claims but helpful for understanding the present invention, and which is purposed to the stimulation of a patient's internal organs or gastrointestinal system. The plurality of stimulation electrodes 1401 are shown here incorporated into a harness and distributed over the patient's torso and abdomen. In other possible embodiments these stimulation electrodes 1401 can just as easily be incorporated into other forms of wearable devices such as vests, shirts, belts, sashes, and girdles which can also focused on covering just the abdomen or just the torso of the patient. The electrical leads 1402 are shown here linking the harness system with the stimulation electrodes to the pulse generation and control electronics 1403. These electrical leads 1402 may be externally visible on the harness or equivalent wearable system or else they may be directly incorporated into the harness or equivalent wearable system and hence only internal to it, or else the harness or equivalent wearable system incorporates a mixture of the two approaches. In all possible embodiments the electrodes can be dual stimulation and sensing electrodes in addition to purely stimulation electrodes, or a mixture of the two types. As such, this approach includes various embodiments intended for various specific uses such as seatbelts or other transportation and safety harness systems where the prompt treatment of a patient cannot be easily facilitated by a third party and hence an in-built capability is required.

The multi-vector patient contact interface described in this document may be placed onto a body of a patient and may be used, for example, to sense the heartbeat of the patient and then deliver a therapeutic pulse to the patient for defibrillation for example. The multi-vector patient contact interface may also be used to deliver other types of treatments of varying energies and durations to the patient. The multi-vector patient contact interface may also be used to sense a characteristic of the patient, such as a heartbeat or pulse and the like. The multi-vector patient contact interface may also be used to both sense a characteristic of the patient and deliver a treatment to the patient when the embodiment of the multi-vector patient contact interface makes use of both sensors and electrodes.

The multi-vector patient contact interface may be placed onto the body of the patient at various locations, such as the torso, limbs and/or head of the patient. In some implementations, multiple multi-vector patient contact interface may be used and each multi-vector patient contact interface may be placed on one or more locations on the body of the patient.

The systems described above may be used to deliver a pulse waveform to a patient using the patient contacts. An example of the pulse generator and waveforms that may be used with the systems are described in co-pending patent application serial numbers 14/303,541 filed on June 12, 2014, 14/661,949 filed March 18, 2015 and 14/662,137 filed March 18, 2015. The one or more multi-vector patient contact interfaces may be attached to a patient and a medical device capable of generating a pulse waveform may be connected to the electrical leads that are connected to the one or more multi-vector patient contact interfaces. A pulse waveform may be delivered from the medical device to the patient via the one or more multi-vector patient contact interfaces. The pulse waveform may be delivered through the one or more multi-vector patient contact interfaces via one or more specific shock vectors (due to the placement of the multi-vector patient contact interfaces on the body of
the patient) and these shock vectors may be selected either statically or dynamically by one or more of the operator of the medical device, a manufacturer of the medical device or an algorithm within the programming of the medical device. In the above delivery of the one or more shock vectors, the shock vector may be a path from one-electrode-to-one-electrode, a path from one-electrode-to-many-electrodes, a path from many-electrodes-to-one-electrode and a path from many-electrodes-to-many-electrodes.

The pulse waveform above may be a multiphasic pulse waveform as described above. The one or more phases of the multiphasic pulse waveform may be each routed via the same selected shock vector. Alternatively, the one or more phases of a multiphasic pulse waveform may be each routed via different selected shock vectors. In some embodiments, the one or more phases of a multiphasic pulse waveform may be each routed via a combination of the same selected shock vector and different selected shock vectors.

The above described embodiments allow for the individual pulses or pulse phases to be delivered in a manner that overlaps in the timing to a greater or lesser degree. In some embodiments, each phase of the multiphasic pulse may be delivered within its own unique segment of the overall pulse timing sequence. In some embodiments, the one or more phases of the multiphasic pulse may be delivered within a time segment that overlaps to a greater or lesser degree with one or more of the other timing segments in the overall pulse sequence.

While the foregoing has been with reference to a particular embodiment of the invention, it will be appreciated by those skilled in the art that changes in this embodiment may be made without departing from the principles of the disclosure, the scope of which is defined by the appended claims.

## Claims

1. A multi-vector patient contact interface, comprising:
a first electrode pad (300) and a second electrode pad (300);
the first and second electrode pad (300) each having a non-conductive substrate (304),
two or more non-conductive adhesive regions (302) formed on the non-conductive substrate (304) and at least a first conductive patient contact (301) and a second conductive patient contact (301) wherein at least one patient contact (301) is adhered to and surrounded by each non-conductive adhesive region (302);
two or more electrical leads (303), wherein each conductive patient contact (301) is electrically connected to a different electrical lead (303);
wherein each conductive patient contact (301) on each electrode pad (300) is electrically isolated from the other conductive patient contacts (301) on the same electrode pad (300) by the non-conductive substrate (304) and the adhesive regions (302);
wherein the first conductive patient contact (301) and the electrical lead (303) connected to the first conductive patient contact (301) of the first electrode pad (300) forms a separate shock vector with the first conductive patient contact (301) and the electrical lead (303) connected to the first conductive patient contact (301) of the second electrode pad (300) and the second conductive patient contact (301) and the electrical lead (303) connected to the second conductive patient contact (301) of the first electrode pad (300) forms a separate shock vector with the second conductive patient contact (301) and the electrical lead (303) connected to the second conductive patient contact (301) of the second electrode pad (300) for a defibrillation pulse; and
wherein each conductive patient contact (301) is adhesive.

2. The interface of Claim 1, wherein the non-conductive substrate (304) is flexible and capable of providing optimal contact with an uneven contact surface when the patient contact interface is placed against the uneven contact surface.

3. The interface of claim 1, wherein each conductive patient contact has a particular shape or wherein the two or more conductive patient contacts (301) each have a variety of shapes or wherein the one or more conductive patient contacts (301) have one or more different sizes.

4. The interface of claim 1, wherein at least one of the two or more conductive patient contacts (301) is a sensor and wherein the sensor is one of an active in nature sensor and a passive in nature sensor.

5. The interface of claim 1, wherein each conductive patient contact (301) is an electrode.

6. The interface of claim 1, wherein the one or more patient contacts (301) are one or more sensors and two or more electrodes.

7. The interface of claim 6, wherein the one or more sensors are arranged in the configuration separately from the two or more electrodes or wherein the one or more sensors are arranged in the configuration intermixed with the two or more electrodes.

8. The interface of claim 1 further comprising a generator that generates a multiphasic pulse and wherein each phase of the multiphasic pulse is delivered within its own unique segment of the overall pulse timing sequence or further comprising a generator that generates a multiphasic pulse and wherein one or more phases of the multiphasic pulse is delivered within a time segment that overlaps to a greater or lesser degree with one or more of the other timing segments in the overall pulse sequence.

## Patentansprüche

1. Eine Multivektor-Patientenkontaktschnittstelle, umfassend:
ein erstes Elektrodenkissen (300) und ein zweites Elektrodenkissen (300);
wobei das erste und das zweite Elektrodenkissen (300) jeweils ein nicht leitfähiges Substrat (304) aufweisen,
zwei oder mehr nicht leitfähige adhäsive Regionen (302), die auf dem nicht leitfähigen Substrat (304) gebildet sind, und mindestens einen ersten leitfähigen Patientenkontakt (301) und einen zweiten leitfähigen Patientenkontakt (301), wobei mindestens ein Patientenkontakt (301) an jeder nicht leitfähigen adhäsiven Region (302) haftet und von dieser umgeben ist;
zwei oder mehr elektrische Leitungen (303), wobei jeder leitfähige Patientenkontakt (301) elektrisch mit einer unterschiedlichen elektrischen Leitung (303) verbunden ist;
wobei jeder leitfähige Patientenkontakt (301) auf jedem Elektrodenkissen (300) von den anderen leitfähigen Patientenkontakten (301) auf demselben Elektrodenkissen (300) durch das nicht leitfähige Substrat (304) und die adhäsiven Regionen (302) elektrisch isoliert ist;
wobei der erste leitfähige Patientenkontakt (301) und die elektrische Leitung (303), die mit dem ersten leitfähigen Patientenkontakt (301) des ersten Elektrodenkissens (300) verbunden ist, einen separaten Schockvektor mit dem ersten leitfähigen Patientenkontakt (301) und der elektrischen Leitung (303) bildet, die mit dem ersten leitfähigen Patientenkontakt (301) des zweiten Elektrodenkissens (300) verbunden ist, und der zweite leitfähige Patientenkontakt (301) und die elektrische Leitung (303), die mit dem zweiten leitfähigen Patientenkontakt (301) des ersten Elektrodenkissens (300) verbunden ist, einen separaten Schockvektor mit dem zweiten leitfähigen Patientenkontakt (301) und der elektrischen Leitung (303) bildet, die mit dem zweiten leitfähigen Patientenkontakt (301) des zweiten Elektrodenkissens (300) für einen Defibrillationsimpuls verbunden ist; und
wobei jeder leitfähige Patientenkontakt (301) adhäsiv ist.

2. Schnittstelle nach Anspruch 1, wobei das nicht leitfähige Substrat (304) flexibel und in der Lage ist, einen optimalen Kontakt mit einer unebenen Kontaktoberfläche bereitzustellen, wenn die Patientenkontaktschnittstelle gegen die unebene Kontaktoberfläche platziert wird.

3. Schnittstelle nach Anspruch 1, wobei jeder leitfähige Patientenkontakt eine bestimmte Form hat oder wobei die zwei oder mehr leitfähigen Patientenkontakte (301) jeweils eine Vielzahl von Formen haben oder wobei der eine oder die mehreren leitfähigen Patientenkontakte (301) eine oder mehrere unterschiedliche Größen haben.

4. Die Schnittstelle nach Anspruch 1, wobei mindestens einer der zwei oder mehr leitfähigen Patientenkontakte (301) ein Sensor ist und wobei der Sensor einer von einem in der Natur aktiven Sensor und einem in der Natur passiven Sensor ist.

5. Die Schnittstelle nach Anspruch 1, wobei jeder leitfähige Patientenkontakt (301) eine Elektrode ist.

6. Die Schnittstelle nach Anspruch 1, wobei der eine oder die mehreren Patientenkontakte (301) ein oder mehrere Sensoren und zwei oder mehrere Elektroden sind.

7. Schnittstelle nach Anspruch 6, wobei der eine oder die mehreren Sensoren in der Konfiguration separat von den zwei oder mehreren Elektroden angeordnet sind oder wobei der eine oder die mehreren Sensoren in der Konfiguration mit den zwei oder mehreren Elektroden vermischt angeordnet sind.

8. Die Schnittstelle nach Anspruch 1, ferner umfassend einen Generator, der einen mehrphasigen Puls erzeugt und wobei jede Phase des mehrphasigen Pulses innerhalb eines eigenen, eindeutigen Segments der Gesamtpuls-Timingsequenz abgegeben wird, oder ferner umfassend einen Generator, der einen mehrphasigen Puls erzeugt und wobei eine oder mehrere Phasen des mehrphasigen Pulses innerhalb eines Zeitsegments abgegeben werden, das sich mehr oder weniger stark mit einem oder mehreren der anderen Timing-Segmente in der Gesamtpulssequenz überlappt.

## Revendications

1. Une interface multi-vectorielle de contact avec un patient, comprenant :
un premier coussinet formant électrode (300) et un deuxième coussinet formant électrode (300) ;
le premier et le deuxième coussinets formant électrodes (300) ayant chacun un substrat non conducteur (304),
deux ou plusieurs zones adhésives (302) non conductrices formées sur le substrat non conducteur (304) et au moins un premier contact de patient conducteur (301) et un deuxième contact de patient conducteur (301), au moins un contact de patient (301) étant lié par adhérence à chaque zone adhésive (302) non conductrice et étant entouré par celle-ci ;
deux fils électriques (303) ou plus, chaque contact de patient conducteur (301) étant électriquement connecté à un fil électrique différent (303) ;
chaque contact de patient conducteur (301) sur chaque électrode (300) étant isolé électriquement des autres contacts conducteurs (301) de patient sur la même électrode (300) par le substrat non conducteur (304) et les zones adhésives (302) ;
le premier contact de patient conducteur (301) et le fil électrique (303) connecté au premier contact de patient conducteur (301) du premier coussinet formant électrode (300) formant un vecteur de choc séparé avec le premier contact de patient conducteur (301) et le fil électrique (303) connecté au premier contact de patient conducteur (301) du deuxième coussinet formant électrode (300) et le deuxième contact de patient conducteur (301) et le fil électrique (303) connecté au deuxième contact de patient conducteur (301) de la première électrode (300) formant un vecteur de choc séparé avec le deuxième contact de patient conducteur (301) et le fil électrique (303) connecté au deuxième contact de patient conducteur (301) de la deuxième électrode (300) pour une impulsion de défibrillation ; et
chaque contact de patient conducteur (301) étant adhésif.

2. L'interface selon la revendication 1, dans laquelle le substrat non conducteur (304) est flexible et apte à fournir un contact optimal avec une surface de contact inégale lorsque l'interface de contact avec le patient est placée contre la surface de contact inégale.

3. L'interface selon la revendication 1, dans laquelle chaque contact conducteur du patient a une forme particulière ou dans laquelle les deux ou plusieurs contacts conducteurs (301) de patient ont chacun une variété de formes ou dans laquelle lesdits un ou plusieurs contacts conducteurs (301) de patient ont une ou plusieurs tailles différentes.

4. L'interface selon la revendication 1, dans laquelle au moins l'un des deux ou plusieurs contacts conducteurs (301) de patient est un capteur et dans laquelle le capteur est l'un parmi un capteur de nature active et un capteur de nature passive.

5. L'interface selon la revendication 1, dans laquelle chaque contact de patient conducteur (301) est une électrode.

6. L'interface selon la revendication 1, dans laquelle lesdits un ou plusieurs contacts de patient (301) sont un ou plusieurs capteurs et deux électrodes ou plus.

7. L'interface selon la revendication 6, dans laquelle lesdits un ou plusieurs capteurs sont agencés dans une configuration de séparation desdites deux ou plusieurs électrodes ou dans laquelle lesdits un ou plusieurs capteurs sont agencés dans une configuration d'entremêlement desdites deux ou plusieurs électrodes.

8. L'interface selon la revendication 1, comprenant en outre un générateur qui génère une impulsion multiphasique et dans lequel chaque phase de l'impulsion multiphasique est délivrée dans son propre segment unique de la séquence de synchronisation d'impulsions globale ou comprenant en outre un générateur qui génère une impulsion multiphasique et dans lequel une ou plusieurs phases de l'impulsion multiphasique sont délivrées dans un segment de temps qui est en chevauchement selon un degré plus ou moins grand avec un ou plusieurs des autres segments de synchronisation dans la séquence d'impulsions globale.
